# EUROPEAN PATENT APPLICATION

(11) **EP 4 227 288 A1**
(43) Date of publication of application: **16.08.2023**
(21) Application number: 21877695.3
(22) Date of filing: 07.10.2021
(51) Int. Cl.: C07C 43/188, C07C 43/192, C07C 43/21, C07C 43/225, C07C 211/36, C07C 211/38, C07C 233/79, C07C 255/54, C07C 255/57, C07C 255/58, C09K 11/06, C07C 217/56, H05B 33/02, H01L 51/50, C07C 69/76

(54) **ADAMANTANE COMPOUND, ORGANIC ELECTROLUMINESCENT ELEMENT AND ELECTRONIC DEVICE**

(30) Priority: 09.10.2020 JP 2020170941
(71) Applicant: Hodogaya Chemical Co., Ltd., Tokyo, 105-0021 (JP)
(72) Inventor: YAMAMOTO, Takeshi, Tokyo 105-0021 (JP); HIRAYAMA, Yuta, Tokyo 105-0021 (JP); HAYASHI, Shuichi, Tokyo 105-0021 (JP)
(74) Representative: Müller-Boré & Partner Patentanwälte PartG mbB
(86) International application number: PCT/JP2021/037089
(87) International publication number: WO 2022/075396

(57) **Abstract**

[Problem]

An object of the present invention is to provide a compound suitable for a low refractive index layer in a capping layer in order to improve the light extraction efficiency of an organic EL device.

[Solution]

The present invention was achieved by focusing on the fact that an adamantane compound has excellent thin film stability and finding that an amide compound, an ester compound, an amine compound, or an ether compound, in which adamantane is arranged at the center, exhibits a low refractive index property, and an organic EL device having excellent luminous efficiency was obtained by using this compound as a material for forming a capping layer having a low refractive index.

## Description

### Technical Field

The present invention relates to an organic electroluminescent device (hereinafter abbreviated as organic EL device) which is a self-luminous device suitable for various display devices, or a compound and a device suitable for an electronic apparatus, and specifically relates to an adamantane compound, and an organic EL device or an electronic apparatus using the compound.

### Background Art

In 1987, C. W. Tang et al. at Eastman Kodak developed a laminated structure device in which materials are assigned with different roles, thereby realizing practical applications of an organic EL device using organic materials. They laminated a phosphor capable of transporting electrons and an organic substance capable of transporting holes, and injected both charges into a phosphor layer to cause emission so as to obtain a high luminance of 1,000 cd/m² or more at a voltage of 10 V or less (see PTLs 1 and 2).

Recently, a light emitting device with a top emission structure in which a metal having a high work function is used for an anode and light is emitted from the top is coming into use. In a bottom emission structure where light is extracted from the bottom having a pixel circuit, the area of a light emitting portion is limited, whereas in the light emitting device with a top emission structure, light is extracted from the top and therefore is not blocked by a pixel circuit, and thus, it has an advantage of having a large emitting portion. In the light emitting device with a top emission structure, a translucent electrode made of LiF/Al/Ag (see, for example, NPL 1), Ca/Mg (see NPL 2), LiF/MgAg, or the like is used for a cathode.

In such a light emitting device, in a case where light emitted by a light emitting layer is incident on another film, when the light is incident at a certain angle or more, the light is totally reflected at an interface between the light emitting layer and the other film. For this reason, only part of the emitted light could be used. Recently, in order to improve the light extraction efficiency, a light emitting device provided with a "capping layer" with a high refractive index on the outside of a translucent electrode with a low refractive index has been proposed (see, for example, NPLs 1 and 2).

On the other hand, an organic optical device, in which a multilayer film that performs effective light propagation control by utilizing the effect of light interference is formed by forming a low refractive index layer through codepositing an additive and laminating it with a high refractive index layer, is shown (see PTL 3).

It is known that the light extraction efficiency of an organic EL device is improved by forming a capping layer having a high refractive index. However, in order to form a low refractive index layer, co-deposition with an additive is performed as described in PTL 3, and there is a demand for a compound that can be vapor-deposited at a low temperature by itself.

### Citation List

### Patent Literature

PTL 1: JPH08-048656A
PTL 2: JP3194657B
PTL 3: JP6210473B

### Non Patent Literature

NPL 1: Appl. Phys. Let., 78, 544 (2001)
NPL 2: Appl. Phys. Let., 82, 466 (2003)

### Summary of Invention

### Technical Problem

An object of the invention is to provide a compound suitable for a low refractive index layer in a capping layer in order to improve the light extraction efficiency of an organic EL device.

Physical properties of a material of the low refractive index layer suitable for the invention include that (1) the material can be vapor-deposited and does not thermally decompose, (2) the thin film state is stable, and (3) the refractive index is low. In addition, physical properties of a device suitable for the invention include that (1) the light extraction efficiency is high, (2) the color purity does not decrease, (3) it transmits light without change over time, and (4) the lifetime is long.

### Solution to Problem

In order to achieve the object, the present inventors focused on the fact that an adamantane compound has excellent thin film stability, and found that an amide compound, an ester compound, an amine compound, or an ether compound, in which adamantane is arranged at the center, exhibits a low refractive index property, and then produced an organic EL device using the compound as a material for forming a capping layer having a low refractive index and diligently evaluated the properties of the device. As a result, they completed the invention.

That is, according to the invention, the following adamantane compounds and organic EL devices are provided.
1) An adamantane compound represented by the following general formula (1). 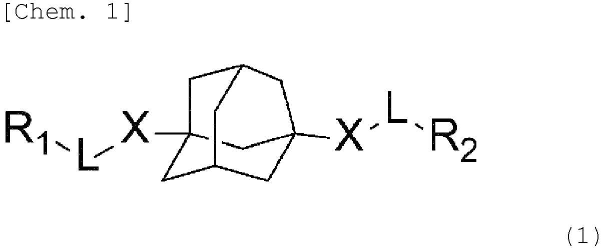 In the formula, X represents an oxygen atom or an NH group, L represents a linear or branched alkylene group having 1 to 3 carbon atoms or a carbonyl group, R₁ and R₂ may be the same or different, and represent a substituted or unsubstituted aromatic hydrocarbon group.
2) The adamantane compound described in the above 1), wherein the adamantane compound is represented by the following general formula (1-A). 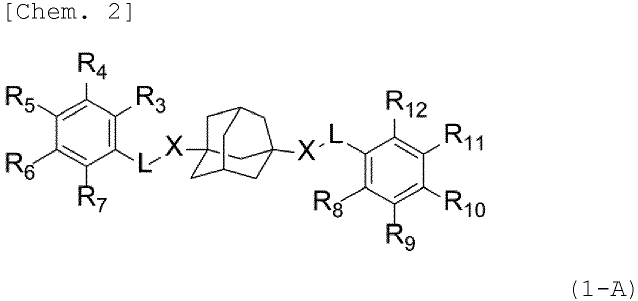 In the formula, X and L are as defined in the general formula (1), R₃ to R₁₂ may be the same or different, and represent a hydrogen atom, a halogen atom, a cyano group, a substituted or unsubstituted linear or branched alkyl group having 1 to 3 carbon atoms, a substituted or unsubstituted linear or branched alkyloxy group having 1 to 3 carbon atoms, or a substituted or unsubstituted aromatic hydrocarbon group.
3) The adamantane compound described in the above 1) or 2), wherein the adamantane compound is represented by the following general formula (1-B). 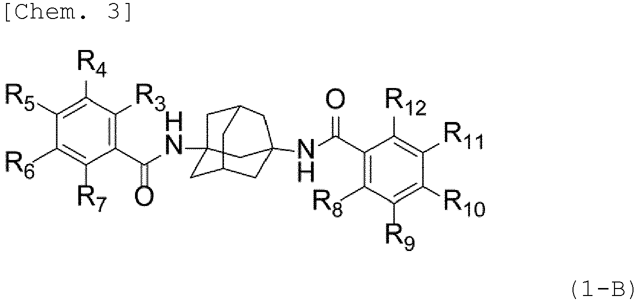 In the formula, R₃ to R₁₂ are as defined in the general formula (1-A).
4) The adamantane compound described in the above 1) or 2), wherein the adamantane compound is represented by the following general formula (1-C). 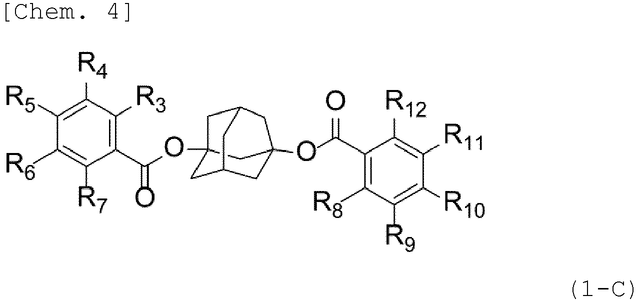 In the formula, R₃ to R₁₂ are as defined in the general formula (1-A).
5) The adamantane compound described in the above 1) or 2), wherein the adamantane compound is represented by the following general formula (1-D). 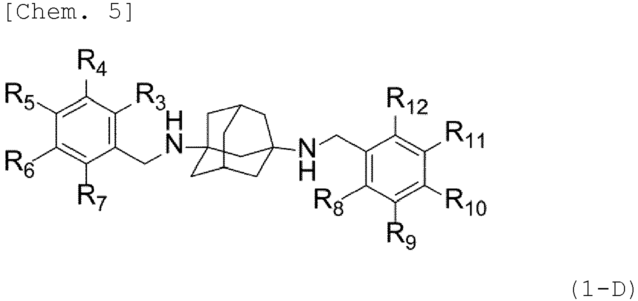 In the formula, R₃ to R₁₂ are as defined in the general formula (1-A).
6) The adamantane compound described in the above 1) or 2), wherein the adamantane compound is represented by the following general formula (1-E). 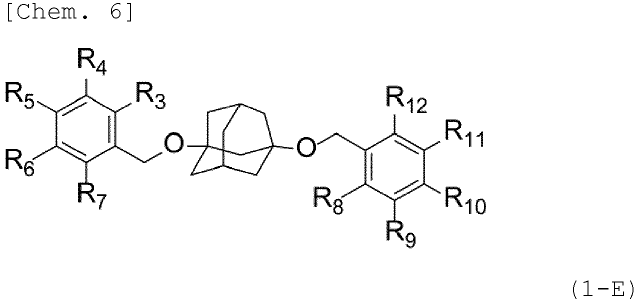 In the formula, R₃ to R₁₂ are as defined in the general formula (1-A).
7) An organic thin film containing the adamantane compound described in any one of the above 1) to 6), characterized by having a refractive index in a wavelength range of 400 nm to 700 nm of 1.60 or less.
8) An organic EL device including at least an anode electrode, a hole transport layer, a light emitting layer, an electron transport layer, a cathode electrode, and a capping layer in this order, characterized in that the capping layer is the organic thin film described in the above 7).
9) An organic EL device including at least an anode electrode, a hole transport layer, a light emitting layer, an electron transport layer, a cathode electrode, and a capping layer in this order, characterized in that the capping layer has a two-layer structure of a first capping layer and a second capping layer, and the first capping layer is the organic thin film described in the above 7).
10) The organic EL device described in the above 9), characterized in that a difference between the refractive index of the first capping layer and the refractive index of the second capping layer ([the refractive index of the second capping layer] - [the refractive index of the first capping layer]) is 0.2 or more.
11) An electronic apparatus or an electronic device including a pair of electrodes and at least one organic layer sandwiched therebetween, characterized in that the adamantane compound described in any one of the above 1) to 6) is used for the organic layer as a constituent material thereof.

In the present specification, the "unsubstituted" in the case of "substituted or unsubstituted" means that a hydrogen atom is not substituted with a substituent.

In the present specification, the "hydrogen atom" is used in the sense of including isotopes having a different number of neutrons, that is, protium and deuterium.

Specific examples of the "alkylene group" in the "linear or branched alkylene group having 1 to 3 carbon atoms" represented by L in the general formula (1) include a methylene group, a 1,2-ethylene group, and a 1,3-propylene group, and a methylene group and a 1,2-ethylene group are preferred, and a methylene group is more preferred.

Further, these divalent groups are preferably unsubstituted, but may have a substituent, and specific examples of the substituent in this case include a cyano group; halogen atoms such as a fluorine atom, a chlorine atom, a bromine atom, and an iodine atom; and linear or branched alkyloxy groups having 1 to 3 carbon atoms such as a methyloxy group, an ethyloxy group, and a propyloxy group.

X in the general formula (1) represents an oxygen atom or an NH group.

Specifically, when X in the general formula (1) is an oxygen atom and L is an alkylene group, (-L-X-) in the general formula (1) is an ether group, and when X in the general formula (1) is an oxygen atom and L therein is a carbonyl group, (-L-X-) in the general formula (1) is an ester group.

When X in the general formula (1) is an NH group and L therein is an alkylene group, (-L—X—) in the general formula (1) is an amino group, and when X in the general formula (1) is an NH group and L therein is a carbonyl group, (-L-X-) in the general formula (1) is an amide group.

Specific examples of the "aromatic hydrocarbon group" in the "substituted or unsubstituted aromatic hydrocarbon group" represented by R₁ and R₂ in the general formula (1) include a phenyl group, a biphenyl group, a 1-naphthyl group, a 2-naphthyl group, a 2-phenanthrenyl group, a 9-phenanthrenyl group, and a fluorenyl group, and a phenyl group, a biphenyl group, a 1-naphthyl group, a 2-naphthyl group, and a fluorenyl group are preferred, and a phenyl group and a fluorenyl group are more preferred.

Further, these groups may have a substituent, and specific examples of the substituent in this case include a cyano group; halogen atoms such as a fluorine atom, a chlorine atom, a bromine atom, and an iodine atom; linear or branched alkyl groups having 1 to 3 carbon atoms such as a methyl group, an ethyl group, and a propyl group; linear or branched alkyloxy groups having 1 to 3 carbon atoms such as a methyloxy group, an ethyloxy group, and a propyloxy group; and aromatic hydrocarbon groups such as a phenyl group, a biphenyl group, a 1-naphthyl group, a 2-naphthyl group, and a fluorenyl group.

Examples of the "halogen atom" represented by R₃ to R₁₂ in the general formula (1-A) include a fluorine atom, a chlorine atom, a bromine atom, and an iodine atom, and a fluorine atom and a chlorine atom are preferred, and a fluorine atom is more preferred.

Examples of the "alkyl group" in the "substituted or unsubstituted linear or branched alkyl group having 1 to 3 carbon atoms" represented by R₃ to R₁₂ in the general formula (1-A) include a methyl group, an ethyl group, and a propyl group, and a methyl group and an ethyl group are preferred, and a methyl group is more preferred.

Examples of the "alkyloxy group" in the "substituted or unsubstituted linear or branched alkyloxy group having 1 to 3 carbon atoms" represented by R₃ to R₁₂ in the general formula (1-A) include a methoxy group, an ethoxy group, and a propyloxy group, and a methoxy group and an ethoxy group are preferred, and a methoxy group is more preferred.

Specific examples of the "aromatic hydrocarbon group" in the "substituted or unsubstituted aromatic hydrocarbon group" represented by R₃ to R₁₂ in the general formula (1-A) include a phenyl group, a biphenyl group, a 1-naphthyl group, a 2-naphthyl group, a 2-phenanthrenyl group, a 9-phenanthrenyl group, and a fluorenyl group, and a phenyl group, a biphenyl group, a 1-naphthyl group, a 2-naphthyl group, and a fluorenyl group are preferred, a phenyl group and a fluorenyl group are more preferred, and a phenyl group is even more preferred.

Further, these groups may have a substituent, and specific examples of the substituent in this case include a cyano group; halogen atoms such as a fluorine atom, a chlorine atom, a bromine atom, and an iodine atom; linear or branched alkyl groups having 1 to 3 carbon atoms such as a methyl group, an ethyl group, and a propyl group; and linear or branched alkyloxy groups having 1 to 3 carbon atoms such as a methyloxy group, an ethyloxy group, and a propyloxy group.

The "linear or branched alkyl group having 1 to 3 carbon atoms" and the "linear or branched alkyloxy group having 1 to 3 carbon atoms" represented by R₃ to R₁₂ in the general formula (1-A) may have a substituent, and as the "substituent" in this case, a halogen atom is preferred, and a fluorine atom is more preferred.

The adamantane compound represented by the general formula (1) of the invention is preferably an adamantane compound represented by any of the following general formulae (1-A) to (1-E), and more preferably an adamantane compound represented by any of the following general formulae (1-B) to (1-E).

The two L's in the general formula (1) are preferably the same group, but may be different groups.

Further, the two X's in the general formula (1) are preferably the same group, but may be different groups.

Further, in the general formula (1), R₁ and R₂ may be the same or different, but are preferably the same.

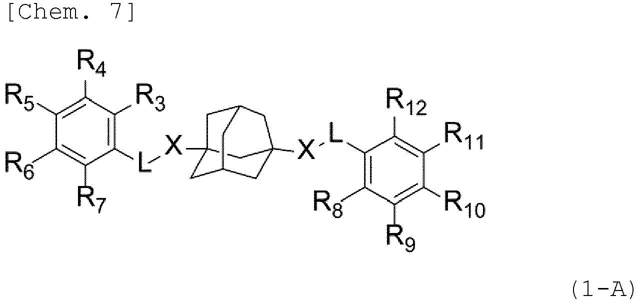

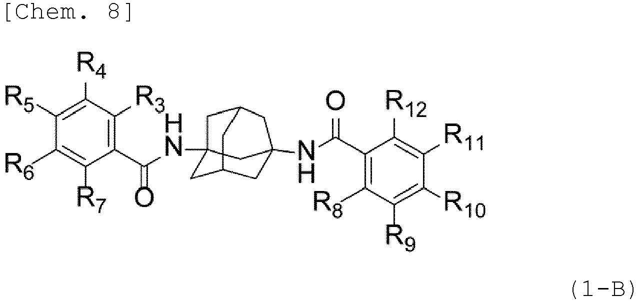

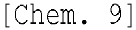

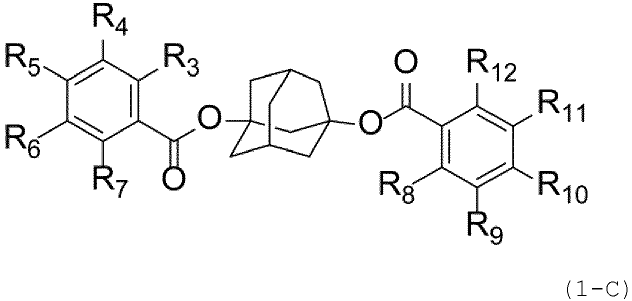

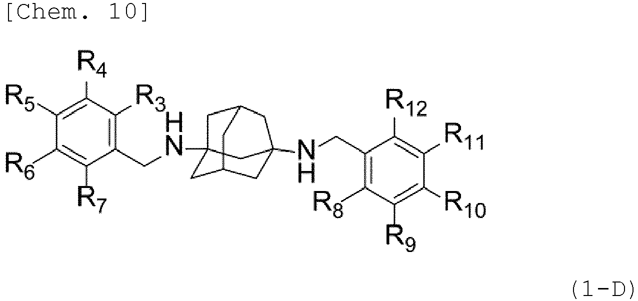

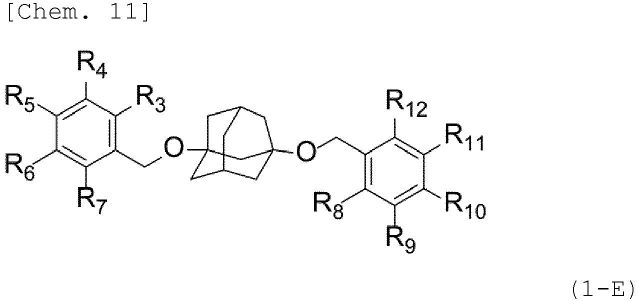

In the general formulae (1-A) to (1-E), X represents an oxygen atom or an NH group, and L represents a linear or branched alkylene group having 1 to 3 carbon atoms or a carbonyl group.

R₃ to R₁₂ may be the same or different, and represent a hydrogen atom, a halogen atom, a cyano group, a substituted or unsubstituted linear or branched alkyl group having 1 to 3 carbon atoms, a substituted or unsubstituted linear or branched alkyloxy group having 1 to 3 carbon atoms, or a substituted or unsubstituted aromatic hydrocarbon group.

The details of the linear or branched alkylene group having 1 to 3 carbon atoms, the aromatic hydrocarbon group, the halogen atom, the linear or branched alkyl group having 1 to 3 carbon atoms, the linear or branched alkyloxy group having 1 to 3 carbon atoms, and the substituent thereof, and the like are as described above.

### Advantageous Effects of Invention

The adamantane compound represented by the general formula (1) of the invention has an excellent low refractive index property. Therefore, by forming a low refractive index layer (organic thin film) using the compound and combining it with a high refractive index layer (organic thin film), an organic EL device having further improved light extraction efficiency due to the effect of light interference is realized.

In addition, the adamantane compound of the invention can be used not only in an organic EL device, but also in the field of electronic apparatus such as an electrophotographic photoreceptor, an image sensor, a photoelectric conversion device, and a solar cell.

### Brief Description of Drawings

[FIG. 1] FIG. 1 is a drawing showing the structures of compounds (1-1) to (1-16) as an adamantane compound represented by the general formula (1) of the invention.
[FIG. 2] FIG. 2 is a drawing showing the structures of compounds (1-17) to (1-32) as the adamantane compound represented by the general formula (1) of the invention.
[FIG. 3] FIG. 3 is a drawing showing the structures of compounds (1-33) to (1-50) as the adamantane compound represented by the general formula (1) of the invention.
[FIG. 4] FIG. 4 is a drawing showing the structures of compounds (1-51) to (1-68) as the adamantane compound represented by the general formula (1) of the invention.
[FIG. 5] FIG. 5 is a drawing showing compounds (1-69) to (1-86) as the adamantane compound represented by the general formula (1) of the invention.
[FIG. 6] FIG. 6 is a drawing showing the structures of compounds (1-87) to (1-104) as the adamantane compound represented by the general formula (1) of the invention.
[FIG. 7] FIG. 7 is a drawing showing the structures of compounds (1-105) to (1-122) as the adamantane compound represented by the general formula (1) of the invention.
[FIG. 8] FIG. 8 is a drawing showing the structures of compounds (1-123) to (1-140) as the adamantane compound represented by the general formula (1) of the invention.
[FIG. 9] FIG. 9 is a drawing showing the structures of compounds (2-1) to (2-10) as specific examples of an arylamine compound having a high refractive index preferably used in an organic EL device of the invention.
[FIG. 10] FIG. 10 is a drawing showing the structures of compounds (2-11) to (2-18) as specific examples of the arylamine compound having a high refractive index preferably used in the organic EL device of the invention.
[FIG. 11] FIG. 11 is a diagram illustrating a configuration of an organic EL device of Examples 6 to 8 and Comparative Examples 1 to 2.

### Description of Embodiments

The adamantane compounds represented by the general formula (1) of the invention are novel compounds, but these compounds per se can be synthesized according to a known method.

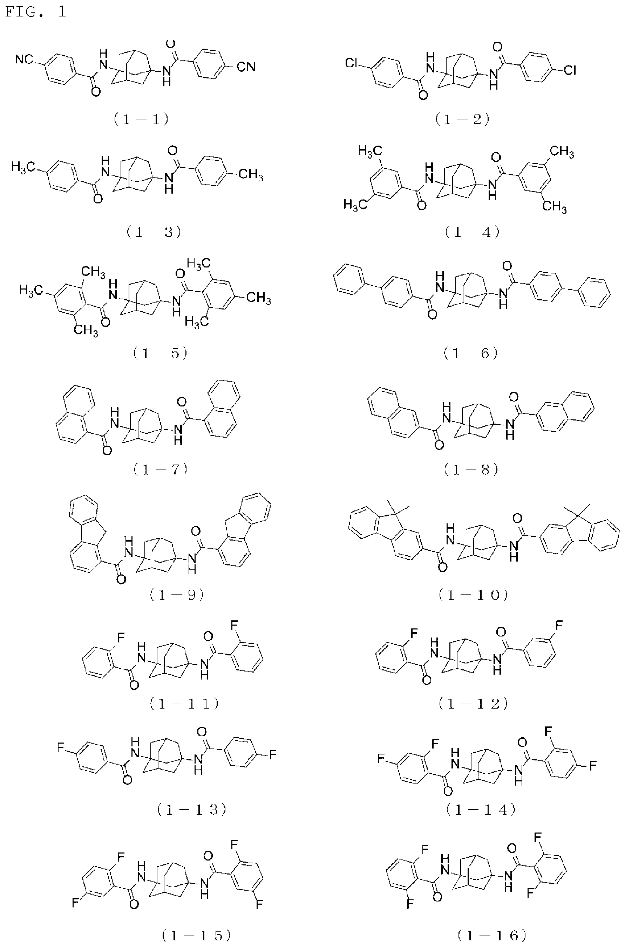

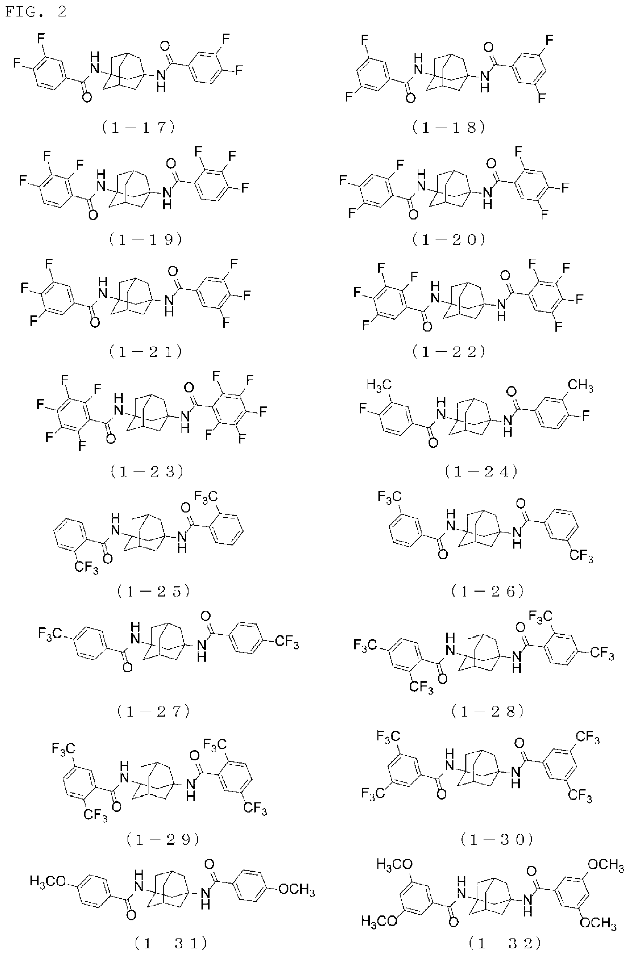

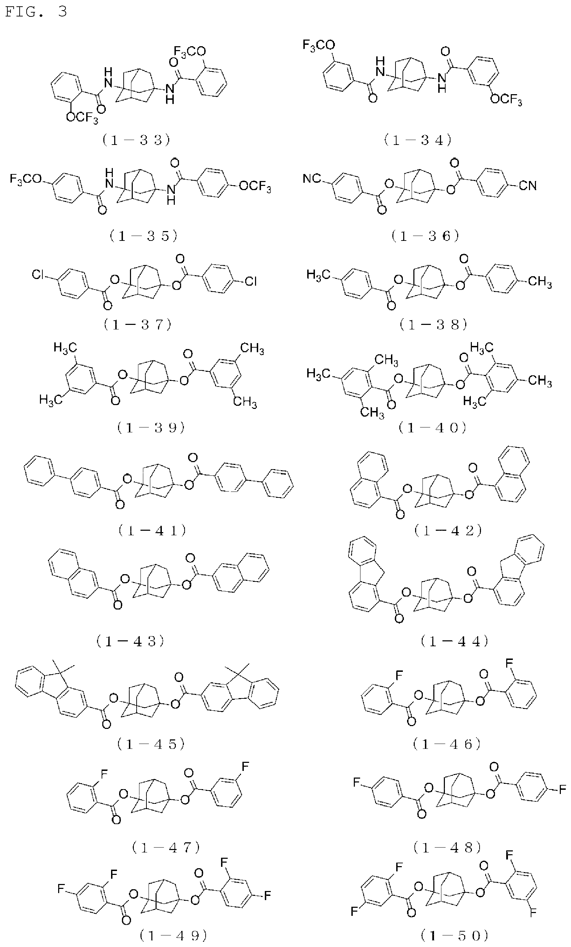

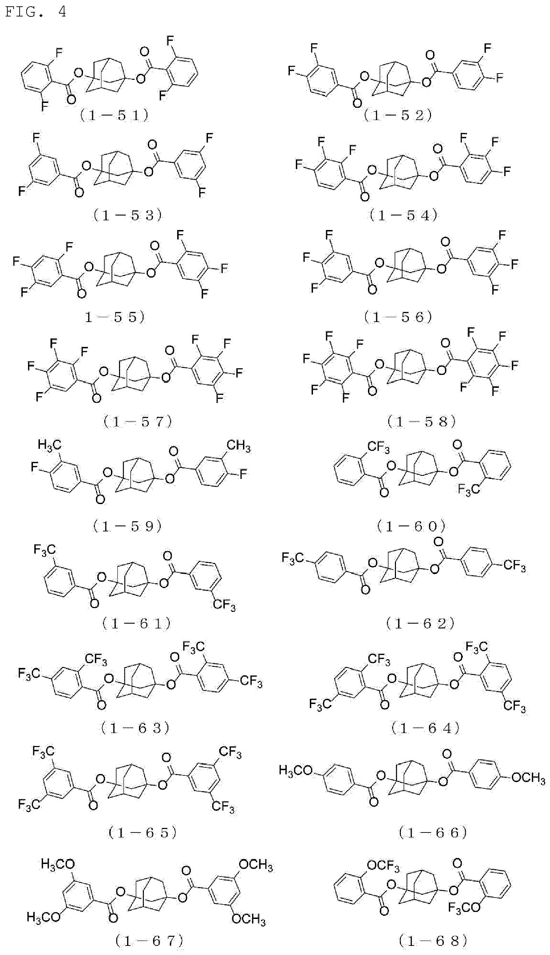

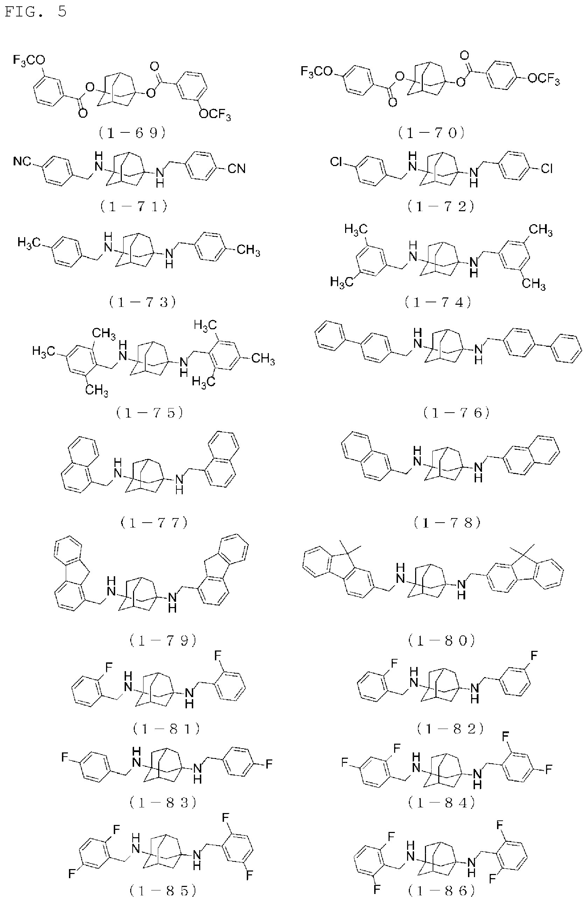

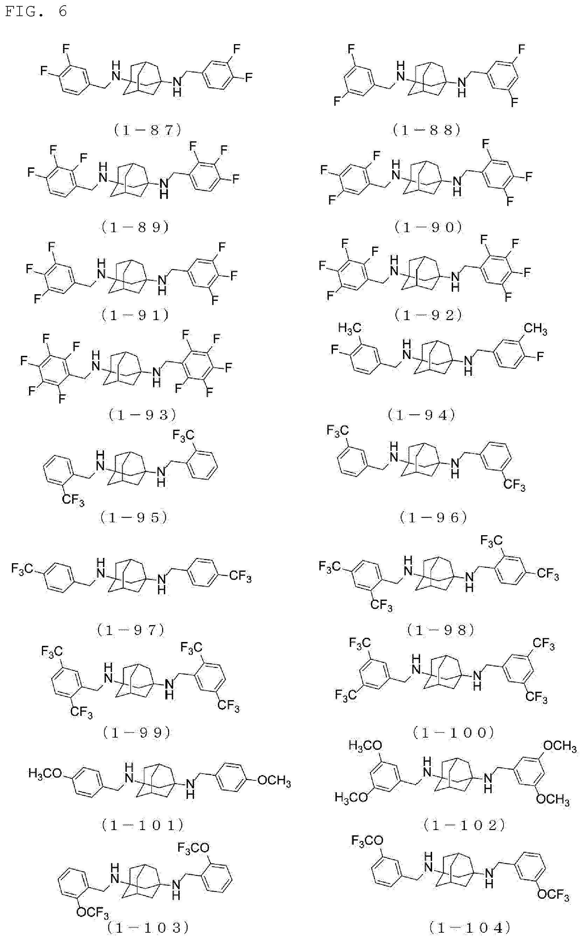

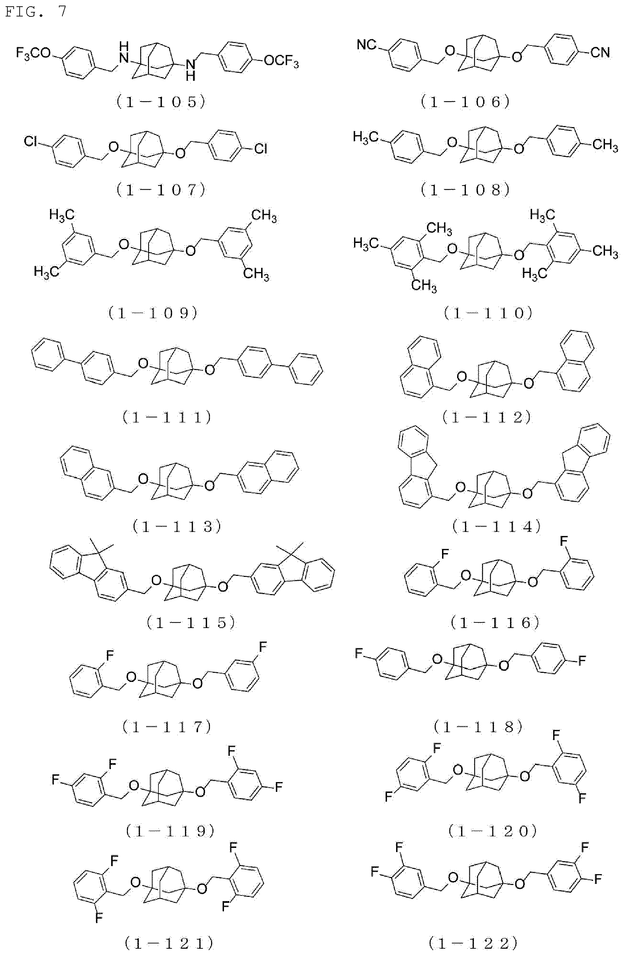

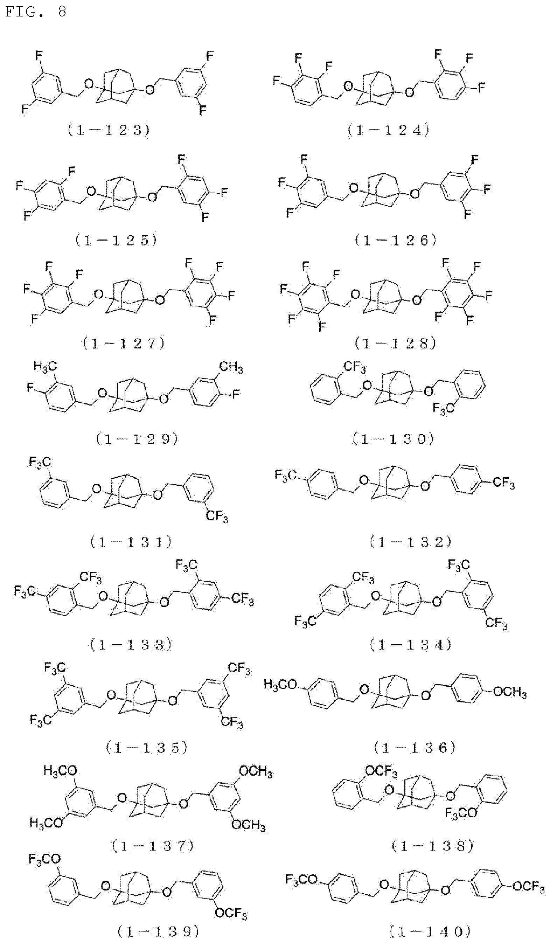

Specific examples of the adamantane compound represented by the general formula (1) of the invention are shown in FIGS. 1 to 8, but the invention is not limited to these compounds.

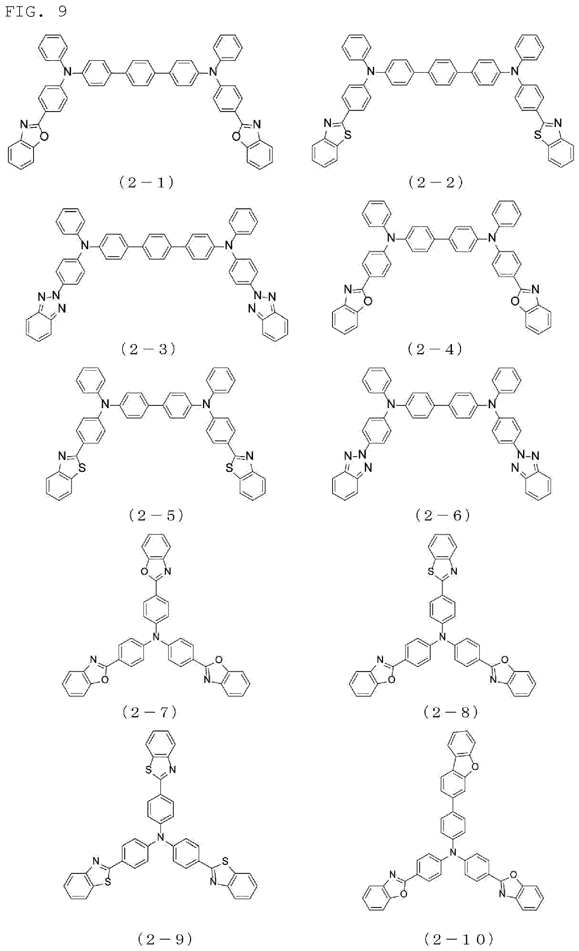

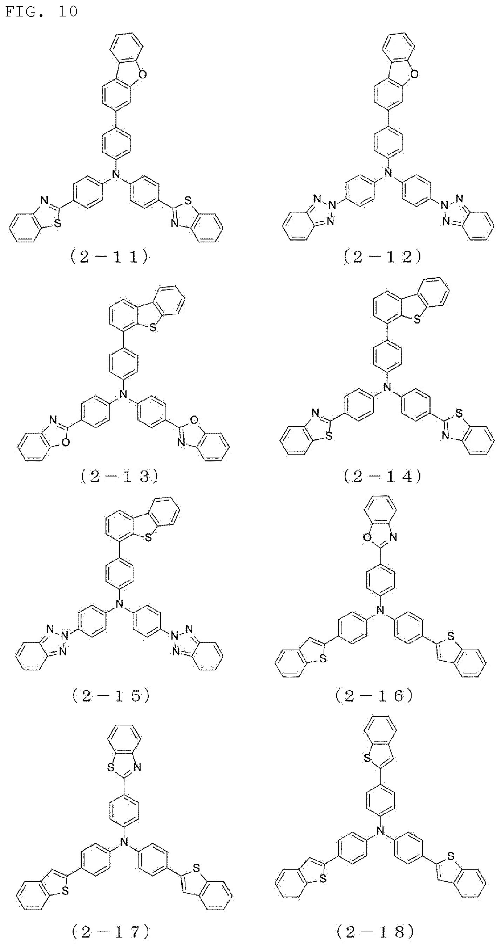

Specific examples of an arylamine compound having a high refractive index, which is preferably used for the organic EL device of the invention, are shown in FIGS. 9 to 10, but the invention is not limited to these compounds.

A method for producing the adamantane compound represented by the general formula (1) of the invention is not particularly limited, but the purification of the compound can be performed by a known method used for purifying an organic compound such as purification using a column chromatograph, adsorption purification using silica gel, activated carbon, activated clay, or the like, recrystallization or crystallization using a solvent, and finally, purification by a sublimation purification method or the like was performed. The identification of the compound can be performed by an NMR analysis, a mass spectrum analysis, or the like. It is preferred to measure a melting point, a glass transition point (Tg), a refractive index, and an absorbance as physical property values.

The melting point and the glass transition point (Tg) were measured by, for example, a high-sensitive differential scanning calorimeter (DSC3100SA manufactured by Bruker AXS) using a powder.

The refractive index was measured using a spectrometer (F10-RT-UV manufactured by Filmetrics) by preparing a thin film of 80 nm on a silicon substrate.

As the structure of the organic EL device of the invention, for example, in the case of a light emitting device with a top emission structure, a structure including an anode, a hole transport layer, a light emitting layer, an electron transport layer, a cathode, and a capping layer sequentially on a glass substrate, and further, a structure including a hole injection layer between the anode and the hole transport layer, a structure including an electron blocking layer between the hole transport layer and the light emitting layer, a structure including a hole blocking layer between the light emitting layer and the electron transport layer, and a structure including an electron injection layer between the electron transport layer and the cathode are exemplified. Some of the organic layers in such a multilayer structure may be omitted or combined, and for example, a configuration in which the hole injection layer and the hole transport layer are combined, a configuration in which the hole transport layer and the electron blocking layer are combined, a configuration in which the hole blocking layer and the electron transport layer are combined, a configuration in which the electron transport layer and the electron injection layer are combined, and so on can be also adopted. Further, a configuration in which two or more organic layers having the same function are laminated can be also adopted, and a configuration in which two hole transport layers are laminated, a configuration in which two light emitting layers are laminated, a configuration in which two electron transport layers are laminated, a configuration in which two capping layers are laminated, and so on can be also adopted.

The total film thickness of the respective layers of the organic EL device is preferably about 200 nm to 750 nm, and more preferably about 350 nm to 600 nm. Further, the film thickness of the capping layer is, for example, preferably 30 nm to 120 nm, and more preferably 40 nm to 80 nm. In this case, good light extraction efficiency is obtained. The film thickness of the capping layer can be appropriately changed according to the type of light emitting material used in the light emitting device, the thickness of each layer of the organic EL device other than the capping layer, or the like.

As the anode of the organic EL device of the invention, an electrode material having a large work function such as ITO or gold is used.

As the hole injection layer of the organic EL device of the invention, materials such as an arylamine compound having a structure in which two or more triphenylamine structures are joined in a molecule via a single bond or a divalent group that does not contain a heteroatom, for example, an arylamine compound having a structure in which two triphenylamine structures are joined in a molecule, such as a benzidine derivative, via a single bond or a divalent group that does not contain a heteroatom, a starburst-type triphenylamine derivative, and various triphenylamine tetramers are preferred. In addition, a porphyrin compound represented by copper phthalocyanine, an accepting heterocyclic compound such as hexacyanoazatriphenylene, or a coating-type polymer material can be used. These may be individually deposited into a film, but may be used as a single layer deposited by being mixed with another material, or may be formed into a laminate structure of individually deposited layers, mixedly deposited layers, or an individually deposited layer and a mixedly deposited layer. These materials can be formed into a thin film by a known method such as a spin coating method or an inkjet method in addition to a vapor deposition method.

As the hole transport layer of the organic EL device of the invention, it is preferred to use a benzidine derivative such as N,N'-diphenyl-N,N'-di(m-tolyl)benzidine (TPD), N,N'-diphenyl-N,N'-di(α-naphthyl)benzidine (NPD), or N,N,N',N'-tetrabiphenylylbenzidine, 1,1-bis[4-(di-4-tolylamino)phenyl]cyclohexane (TAPC), particularly, an arylamine compound having a structure in which two triphenylamine structures are joined in a molecule via a single bond or a divalent group which does not contain a heteroatom, for example, N,N,N',N'-tetrabiphenylylbenzidine or the like. In addition, it is preferred to use an arylamine compound having only one triphenylamine structure in a molecule, an arylamine compound having a structure in which three or more triphenylamine structures are joined in a molecule via a single bond or a divalent group which does not contain a heteroatom, for example, various triphenylamine trimers and tetramers, or the like. These may be individually deposited into a film, but may be used as a single layer deposited by being mixed with another material, or may be formed into a laminate structure of individually deposited layers, mixedly deposited layers, or an individually deposited layer and a mixedly deposited layer. In addition, a coating-type polymer material such as poly(3,4-ethylenedioxythiophene) (PEDOT)/poly(styrene sulfonate) (PSS) can be used as a hole injection/transport layer. These materials can be formed into a thin film by a known method such as a spin coating method or an inkjet method in addition to a vapor deposition method.

Further, in the hole injection layer or the hole transport layer, a material obtained by further p-doping a material which is commonly used for the layer with trisbromophenylamine hexachloroantimony, a radialene derivative, or the like is preferred. In addition, a polymer compound having a structure of a benzidine derivative such as TPD in its partial structure, or the like can be used.

As the electron blocking layer of the organic EL device of the invention, a compound having an electron blocking effect, for example, a carbazole derivative such as 4,4',4''-tri(N-carbazolyl)triphenylamine (TCTA), 9,9-bis[4-(carbazol-9-yl)phenyl]fluorene, 1,3-bis(carbazol-9-yl)benzene (mCP), or 2,2-bis(4-carbazol-9-yl-phenyl)adamantane (Ad-Cz), a compound having a triphenylsilyl group and a triarylamine structure represented by 9-[4-(carbazol-9-yl)phenyl]-9-[4-(triphenylsilyl)phenyl]-9H-fluorene, or the like can be used. These may be individually deposited into a film, but may be used as a single layer deposited by being mixed with another material, or may be formed into a laminate structure of individually deposited layers, mixedly deposited layers, or an individually deposited layer and a mixedly deposited layer. These materials can be formed into a thin film by a known method such as a spin coating method or an inkjet method in addition to a vapor deposition method.

As the light emitting layer of the organic EL device of the invention, in addition to a metal complex of a quinolinol derivative such as Alq₃, various metal complexes, an anthracene derivative, a bisstyrylbenzene derivative, a pyrene derivative, an oxazole derivative, a polyparaphenylene vinylene derivative, or the like can be used. Further, the light emitting layer may be formed of a host material and a dopant material, and as the host material, an anthracene derivative is preferably used. Furthermore, in addition to the light emitting materials, a heterocyclic compound having an indole ring as a partial structure of a fused ring, a heterocyclic compound having a carbazole ring as a partial structure of a fused ring, a carbazole derivative, a thiazole derivative, a benzimidazole derivative, a polydialkylfluorene derivative, or the like can be used. Further, as the dopant material, quinacridone, coumarin, rubrene, perylene, a derivative thereof, a benzopyran derivative, a rhodamine derivative, an aminostyryl derivative, or the like can be used, and it is more preferred to use a green light emitting material. These may be individually deposited into a film, but may be used as a single layer deposited by being mixed with another material, or may be formed into a laminate structure of individually deposited layers, mixedly deposited layers, or an individually deposited layer and a mixedly deposited layer.

Further, as the light emitting material, a phosphorescent material can be also used. As the phosphorescent material, a phosphorescent material of a metal complex such as iridium or platinum can be used. A green phosphorescent material such as Ir(ppy)₃, a blue phosphorescent material such as FIrpic or FIr6, a red phosphorescent material such as Btp₂Ir(acac), or the like is used, and it is more preferred to use a green phosphorescent material. As the host material at that time, a carbazole derivative such as 4,4'-di(N-carbazolyl)biphenyl (CBP), TCTA, or mCP can be used as a hole injecting and transporting host material. As an electron transporting host material, p-bis(triphenylsilyl)benzene (UGH2), 2,2',2"-(1,3,5-phenylene)-tris(1-phenyl-1H-benzimidazole) (TPBI), or the like can be used, and a high-performance organic EL device can be fabricated.

In order to avoid concentration quenching, the doping of the host material with the phosphorescent light emitting material is preferably performed by co-deposition in a range of 1 to 30 wt% with respect to the whole light emitting layer.

Further, as the light emitting material, it is also possible to use a material that emits delayed fluorescence. Such a material can be formed into a thin film by a known method such as a spin coating method or an inkjet method in addition to a vapor deposition method.

As the hole blocking layer of the organic EL device of the invention, a compound having a hole blocking effect, for example, a phenanthroline derivative such as bathocuproine (BCP), a metal complex of a quinolinol derivative such as aluminum(III) bis(2-methyl-8-quinolinate)-4-phenylphenolate (BAlq), various rare earth complexes, a triazole derivative, a triazine derivative, a pyrimidine derivative, an oxadiazole derivative, a benzazole derivative, or the like can be used. These materials may also serve as the material of the electron transport layer. These may be individually deposited into a film, but may be used as a single layer deposited by being mixed with another material, or may be formed into a laminate structure of individually deposited layers, mixedly deposited layers, or an individually deposited layer and a mixedly deposited layer. These materials can be formed into a thin film by a known method such as a spin coating method or an inkjet method in addition to a vapor deposition method.

As the electron transport layer of the organic EL device of the invention, in addition to a metal complex of a quinolinol derivative such as Alq₃ or BAlq, various metal complexes, a triazole derivative, a triazine derivative, a pyrimidine derivative, an oxadiazole derivative, a pyridine derivative, a benzimidazole derivative, a benzazole derivative, a thiadiazole derivative, an anthracene derivative, a carbodiimide derivative, a quinoxaline derivative, a pyridoindole derivative, a phenanthroline derivative, a silole derivative, or the like can be used. These may be individually deposited into a film, but may be used as a single layer deposited by being mixed with another material, or may be formed into a laminate structure of individually deposited layers, mixedly deposited layers, or an individually deposited layer and a mixedly deposited layer. These materials can be formed into a thin film by a known method such as a spin coating method or an inkjet method in addition to a vapor deposition method.

As the electron injection layer of the organic EL device of the invention, an alkali metal salt such as lithium fluoride or cesium fluoride, an alkaline earth metal salt such as magnesium fluoride, a metal complex of a quinolinol derivative such as lithium quinolinol, a metal oxide such as aluminum oxide, or a metal such as ytterbium (Yb), samarium (Sm), calcium (Ca), strontium (Sr), or cesium (Cs), or the like can be used. However, the electron injection layer may be omitted in the preferred selection of the electron transport layer and the cathode.

Further, in the electron injection layer or the electron transport layer, a material obtained by further n-doping a material which is commonly used for the layer with a metal such as cesium can be used.

As the cathode of the organic EL device of the invention, an electrode material having a low work function such as aluminum, or an alloy having a lower work function such as a magnesium-silver alloy, a magnesium-calcium alloy, a magnesium-indium alloy, or an aluminum-magnesium alloy, or ITO, IZO, or the like is used as the electrode material.

The capping layer of the organic EL device of the invention preferably has a two-layer structure of a first capping layer and a second capping layer, and in that case, as the first capping layer adjacent to the cathode electrode, it is preferred to use the adamantane compounds represented by the above general formula (1) of the invention.

These may be individually deposited into a film, but may be used as a single layer deposited by being mixed with another material. These materials can be formed into a thin film by a known method such as a spin coating method or an inkjet method in addition to a vapor deposition method.

The adamantane compound represented by the above general formula (1) of the invention preferably has a refractive index in a wavelength range of 400 nm to 700 nm of 1.6 or less, and more preferably 1.5 or less.

In the organic EL device of the invention, as the second capping layer to be laminated on the first capping layer, it is preferred to use an arylamine compound having a high refractive index. The arylamine compound having a high refractive index preferably has a refractive index in a wavelength range of 450 nm to 700 nm of 1.6 or more, more preferably 1.8 or more, and even more preferably 1.9 or more. As such an arylamine compound having a high refractive index, as illustrated in FIGS. 9 to 10, one having a structure in which two triphenylamine structures are joined in a molecule via a single bond or a phenylene group and having two benzazole groups or benzotriazole groups as substituents, or one having only one triphenylamine structure in a molecule and having two or three benzazole groups, benzotriazole groups, or benzothienyl groups as substituents is preferably used.

These compounds have no absorption in each of the blue, green, and red wavelength regions, and therefore are particularly preferred when a clear and bright image with good color purity is desired to be displayed.

These may be individually deposited into a film, but may be used as a single layer deposited by being mixed with another material. These materials can be formed into a thin film by a known method such as a spin coating method or an inkjet method in addition to a vapor deposition method.

The refractive index of the material for forming the second capping layer is more preferably 0.2 or more larger than the refractive index of the first capping layer adjacent thereto ([the refractive index of the second capping layer] - [the refractive index of the first capping layer] ≧ 0.2). That is, by the second capping layer, the light extraction efficiency in the organic EL device is improved, but the effect thereof is more effective because the larger the reflectance at the interface between the second capping layer and the first capping layer, the larger the effect of light interference. Therefore, it is more preferred that the refractive index of the material for forming the second capping layer is 0.2 or more larger than the refractive index of the first capping layer adjacent thereto.

Although the organic EL device with a top emission structure has been described above, the invention is not limited thereto, and can be similarly applied as well to an organic EL device with a bottom emission structure or an organic EL device with a dual emission structure that emits light from both the top and the bottom. In these cases, the electrode located in the direction in which light is extracted from the light emitting device to the outside needs to be transparent or translucent.

Hereinafter, embodiments of the invention will be more specifically described with reference to Examples. The invention, however, is not limited to the following Examples as long as it does not depart from the gist of the invention.

### Example 1

### <Synthesis of N,N'-adamantane-1,3-diylbis(4-fluorobenzeneamide) (1-13)>

To a reaction vessel purged with nitrogen, 5.0 g of adamantane-1,3-diamine, 120 mL of tetrahydrofuran, and 10.4 g of potassium carbonate were added, and then, 10.5 g of 4-fluorobenzoyl chloride was added thereto dropwise over 10 minutes, and further, the mixture was stirred at 25°C for 4 hours. 100 mL of water was added thereto, and tetrahydrofuran was distilled off under reduced pressure, and then, the precipitated solid was collected by filtration. 60 mL of methanol and 60 mL of water were added thereto, and reflux dispersion washing was performed for 1 hour, followed by cooling to room temperature, and then, the solid was collected by filtration. The solid was dissolved in 180 mL of dichloromethane, and 6 g of silica gel was added thereto, and the mixture was stirred at 25°C for 1 hour, and then, the silica gel was removed by filtration. The filtrate was concentrated, and the residue was washed with methanol, whereby 8.4 g of a white powder of N,N'-adamantane-1,3-diylbis(4-fluorobenzeneamide) (1-13) (yield: 68%) was obtained.

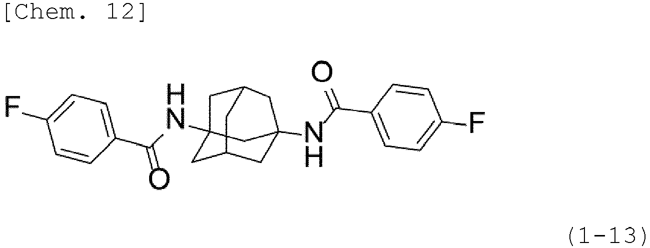

The structure of the obtained white powder was identified using NMR.

The following 24 hydrogen signals were detected by ¹H-NMR (CDCl₃).
δ (ppm) = 7.71-7.74 (4H), 7.06-7.11 (4H), 5.31 (2H), 2.57 (2H), 2.33 (2H), 2.07-2.19 (8H), 1.71 (2H).

### Example 2

### <Synthesis of N,N'-adamantane-1,3-diylbis(3,5-difluorobenzeneamide) (1-18)>

The same operation as in Example 1 was performed except that 4-fluorobenzoyl chloride was replaced with 3,5-difluorobenzoyl chloride, whereby 11.4 g of a white powder of N,N'-adamantane-1,3-diylbis(3,5-difluorobenzeneamide) (1-18) (yield: 80.9%) was obtained.

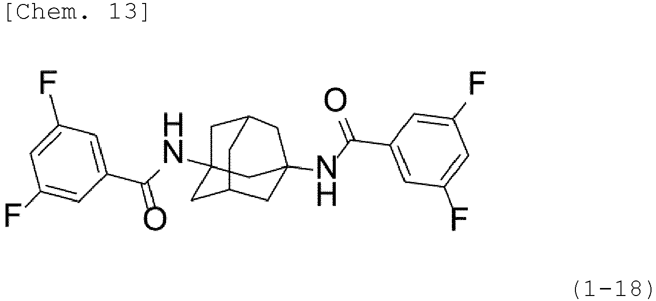

The structure of the obtained white powder was identified using NMR.

The following 22 hydrogen signals were detected by ¹H-NMR (CDCl₃).
δ (ppm) = 7.22-7.29 (4H), 6.92-6.97 (2H), 5.88 (2H), 2.57 (2H), 2.37 (2H), 2.07-2.18 (8H), 1.73 (2H).

### Example 3

### <Synthesis of N,N'-adamantane-1,3-diylbis(pentafluorobenzeneamide) (1-23)>

The same operation as in Example 1 was performed except that 4-fluorobenzoyl chloride was replaced with pentafluorobenzoyl chloride, whereby 9.0 g of a white powder of N,N'-adamantane-1,3-diylbis(pentafluorobenzeneamide) (1-23) (yield: 54%) was obtained.

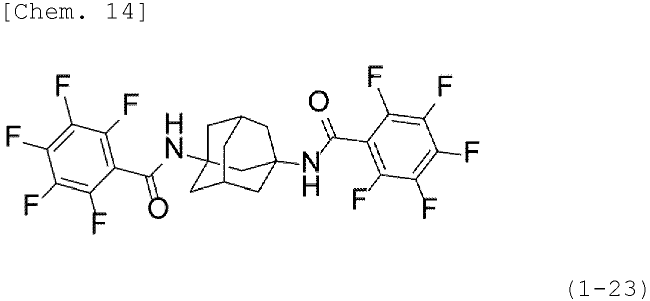

The structure of the obtained white powder was identified using NMR and a mass spectrum analysis.

The following 16 hydrogen signals were detected by ¹H-NMR (DMSO-d₆).
δ (ppm) = 8.64 (2H), 2.36 (2H), 2.23 (2H), 1.93-2.04 (8H), 1.61 (2H).

The following ¹³C signals were detected by ¹³C-NMR (DMSO-d₆).
δ (ppm) = 157.0, 144.6, 142.5, 142.2, 140.0, 138.5, 138.4, 136.2, 136.0, 114.1, 113.9, 113.6, 67.5, 54.1, 54.0, 44.4, 39.4, 35.1, 29.6, 25.6.
m/z (M+1) = 555

### Example 4

The glass transition point (Tg) and the melting point of each of the adamantane compounds represented by the general formula (1) were measured using a high-sensitive differential scanning calorimeter (DSC3100SA manufactured by Bruker AXS).

| Glass transition point (Tg) | | Melting point |
|---|---|---|
| Compound (1-13) of Example 1 | 71°C | 222°C |
| Compound (1-18) of Example 2 | (not observed) | 256°C |
| Compound (1-23) of Example 3 | (not observed) | 322°C |

### Example 5

A vapor-deposited film with a film thickness of 80 nm was fabricated on a silicon substrate using the adamantane compound represented by the general formula (1), and the refractive index n at each of wavelengths of 400 nm, 450 nm, and 700 nm was measured using a spectrometer (F10-RT-UV manufactured by Filmetrics). Further, for comparison, measurement was performed also for an arylamine compound (2-8) having a high refractive index and Alq₃. The measurement results are summarized in Table 1.

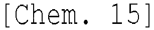

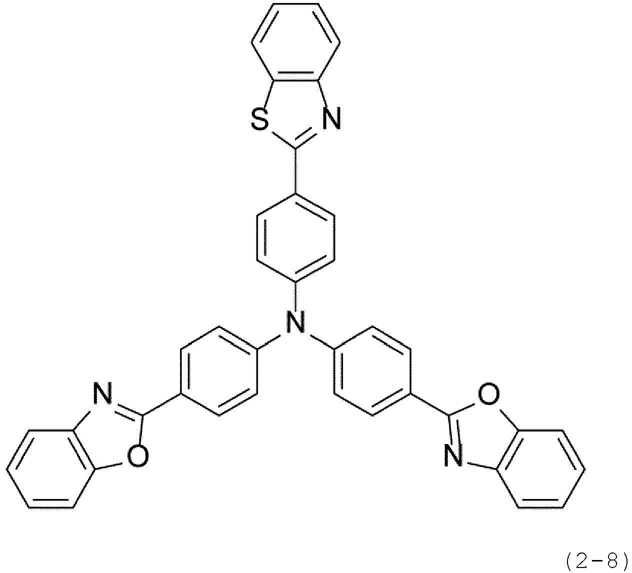

**[Table 1]**

| | Refractive index n (*λ* : 400nm) | Refractive index n (*λ* : 450nm) | Refractive index n (*λ* : 700nm) |
|---|---|---|---|
| Compound(1 - 13) | 1. 42 | 1. 42 | 1.42 |
| Compound (1 - 18) | 1. 25 | 1.27 | 1. 31 |
| Compound (1 -23) | 1. 19 | 1.20 | 1.22 |
| Compound (2 - 8) | 2. 47 | 2. 37 | 1.93 |
| Alq₃ | 1. 86 | 1.88 | 1. 74 |

In this manner, the adamantane compounds represented by the general formula (1) of the invention each have a refractive index in a wavelength range of 400 nm to 700 nm of 1.60 or less, which is 0.2 or more smaller than the refractive index of the arylamine compound (2-8) having a high refractive index.

### Example 6

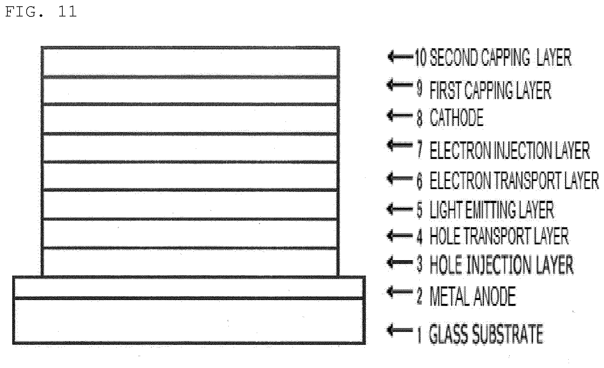

An organic EL device was fabricated by vapor-depositing a hole injection layer 3, a hole transport layer 4, a light emitting layer 5, an electron transport layer 6, an electron injection layer 7, a cathode 8, a first capping layer 9, and a second capping layer 10 in this order on a reflective ITO electrode formed beforehand as a metal anode 2 on a glass substrate 1 as shown in FIG. 11.

Specifically, as the metal anode 2, ITO with a film thickness of 50 nm, a silver alloy reflective film with a film thickness of 100 nm, and ITO with a film thickness of 5 nm were sequentially deposited on the glass substrate 1, and the resultant was subjected to ultrasonic washing for 20 minutes in isopropyl alcohol, and then dried for 10 minutes on a hot plate heated to 250°C. Thereafter, a UV ozone treatment was performed for 2 minutes, and then, the glass substrate with ITO was attached inside a vacuum vapor deposition apparatus, and the pressure was reduced to 0.001 Pa or less. Subsequently, as the hole injection layer 3, an electron acceptor (Acceptor-1) of the following structural formula and a compound (3-1) of the following structural formula were formed to a film thickness of 10 nm by binary vapor deposition at such a vapor deposition rate that the vapor deposition rate ratio is as follows: Acceptor-1 : the compound (3-1) = 3:97 so as to cover the metal anode 2. On the hole injection layer 3, as the hole transport layer 4, the compound (3-1) of the following structural formula was formed to a film thickness of 140 nm. On the hole transport layer 4, as the light emitting layer 5, a compound (EMD-1) of the following structural formula and a compound (EMH-1) of the following structural formula were formed to a film thickness of 20 nm by binary vapor deposition at such a vapor deposition rate that the vapor deposition rate ratio is as follows: the compound (EMD-1) : the compound (EMH-1) = 5:95. On the light emitting layer 5, as the electron transport layer 6, a compound (4-1) of the following structural formula and a compound (ETM-1) of the following structural formula were formed to a film thickness of 30 nm by binary vapor deposition at such a vapor deposition rate that the vapor deposition rate ratio was as follows: the compound (4-1) : the compound (ETM-1) = 50:50. On the electron transport layer 6, as the electron injection layer 7, lithium fluoride was formed to a film thickness of 1 nm. On the electron injection layer 7, as the cathode 8, a magnesium-silver alloy was formed to a film thickness of 12 nm. On the cathode 8, as the first capping layer 9, the compound (1-13) of Example 1 was formed to a film thickness of 30 nm, and finally, as the second capping layer 10, the arylamine compound (2-8) having a high refractive index was formed to a film thickness of 30 nm. The characteristics of the fabricated organic EL device were measured in the air at normal temperature.

The measurement results of the emission characteristics when applying a DC voltage to the fabricated organic EL device are summarized in Table 2.

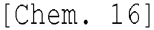

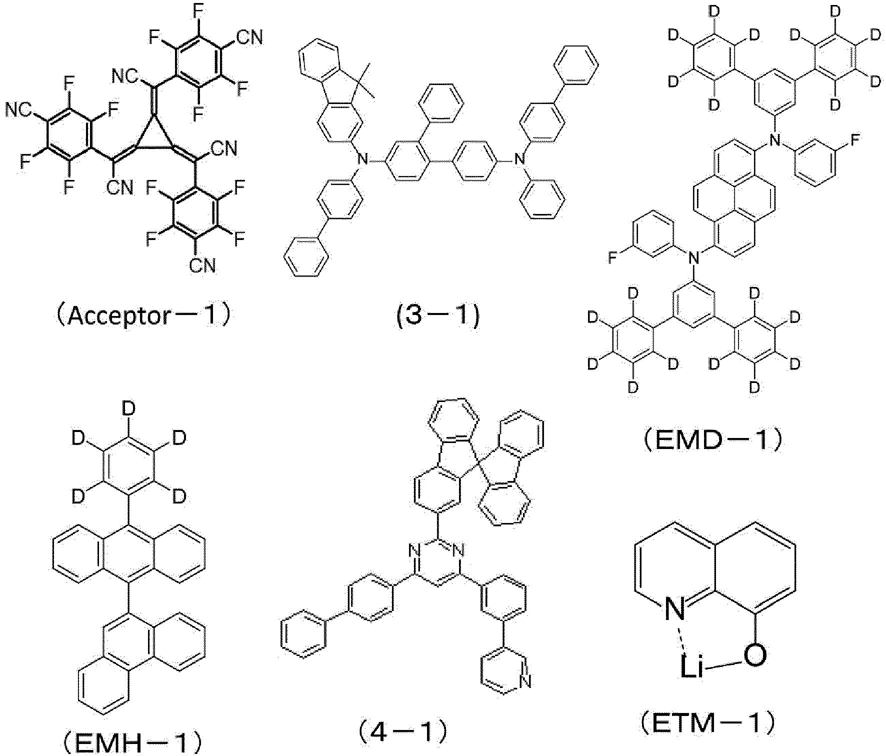

### Example 7

An organic EL device was fabricated under the same conditions as in Example 6 except that as the first capping layer 9, the compound (1-18) of Example 2 was used in place of the compound (1-13) of Example 1. The characteristics of the fabricated organic EL device were measured in the air at normal temperature.

The measurement results of the emission characteristics when applying a DC voltage to the fabricated organic EL device are summarized in Table 2.

### Example 8

An organic EL device was fabricated under the same conditions as in Example 6 except that as the first capping layer 9, the compound (1-23) of Example 3 was used in place of the compound (1-13) of Example 1. The characteristics of the fabricated organic EL device were measured in the air at normal temperature.

The measurement results of the emission characteristics when applying a DC voltage to the fabricated organic EL device are summarized in Table 2.

### [Comparative Example 1]

For comparison, an organic EL device was fabricated under the same conditions as in Example 6 except that as the first capping layer 9, Alq₃ was used in place of the compound (1-13) of Example 1. The characteristics of the fabricated organic EL device were measured in the air at normal temperature.

The measurement results of the emission characteristics when applying a DC voltage to the fabricated organic EL device are summarized in Table 2.

### [Comparative Example 2]

For comparison, an organic EL device in which as the second capping layer 10, the arylamine compound (2-8) having a high refractive index was formed to a film thickness of 60 nm which is twice as large as that in Example 6, and the first capping layer 9 was not provided was fabricated. The characteristics of the fabricated organic EL device were measured in the air at normal temperature.

The measurement results of the emission characteristics when applying a DC voltage to the fabricated organic EL device are summarized in Table 2.

The results of measuring the device lifetime using the organic EL devices fabricated in Examples 6 to 8 and Comparative Examples 1 to 2 are summarized in Table 2. The device lifetime was measured as a time elapsed until the luminance was attenuated to 95% with the initial luminance taken as 100% (attenuation to 95%) when constant current driving at 10 mA/cm² was performed.

**[Table 2]**

| | First capping layer | Second capping layer | Voltage[V] (@10mA/cm²) | Luminance [cd/m²] (@10mA/cm²) | Luminous efficiency [od/A] (@10mA/cm²) | Power efficiency [Im/W] (@10mA/cm²) | Device lifetime Attenuation to 95% |
|---|---|---|---|---|---|---|---|
| Examples | Compound (1-13) | Compound (2-8) | 3.60 | 829 | 8. 29 | 7. 23 | 184h |
| Example 7 | Compound (1-18) | Compound (2-8) | 3.61 | 832 | 8. 32 | 7.25 | 178h |
| Examples | Compound (1-23) | Compound (2-8) | 3. 64 | 778 | 7. 78 | 6. 72 | 192h |
| Comparative Example 1 | Alq₃ | Compound (2-8) | 3. 65 | 752 | 7. 51 | 6. 46 | 168h |
| Comparative Example2 | - | Compound (2-8) | 3. 65 | 708 | 7. 08 | 6. 10 | 159h |

As shown in Table 2, the driving voltage at a current density of 10 mA/cm² was almost the same in the devices of Comparative Examples 1 and 2 and the devices of Examples 6 to 8 using the adamantane compound represented by the general formula (1) of the invention as the first capping layer. However, the luminance, the luminous efficiency, the power efficiency, and the device lifetime were all significantly improved in the devices of Examples 6 to 8 as compared to the devices of Comparative Examples 1 and 2. This indicates that the light extraction efficiency can be significantly improved by forming a laminate structure in which the second capping layer is laminated on the first capping layer containing the adamantane compound represented by the general formula (1) of the invention, and further combining the material of the second capping layer so that the difference in the refractive index between the first capping layer and the second capping layer is large.

### Industrial Applicability

The organic EL device containing the adamantane compound represented by the general formula (1) of the invention in the capping layer, particularly, the organic EL device in which a material that makes the difference in refractive index larger is laminated as the second capping layer on the first capping layer containing the adamantane compound represented by the general formula (1) of the invention can obtain high light extraction efficiency. Further, it is particularly preferred when a clear and bright image with good color purity is desired to be displayed by using the compound having no absorption in each of the blue, green, and red wavelength regions. For example, it has become possible to develop applications to home electric appliances and illuminations.

### Reference Signs List

- 1: Glass substrate
- 2: Metal anode
- 3: Hole injection layer
- 4: Hole transport layer
- 5: Light emitting layer
- 6: Electron transport layer
- 7: Electron injection layer
- 8: Cathode
- 9: First capping layer
- 10: Second capping layer

## Claims

1. An adamantane compound represented by the following general formula (1): 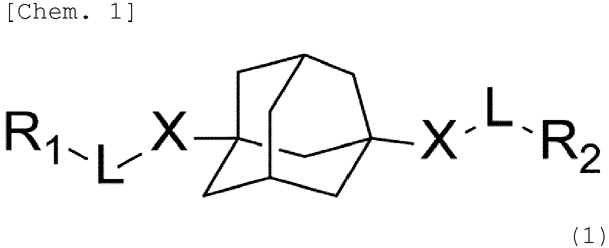 wherein X represents an oxygen atom or an NH group, L represents a linear or branched alkylene group having 1 to 3 carbon atoms or a carbonyl group, R₁ and R₂ may be the same or different, and represent a substituted or unsubstituted aromatic hydrocarbon group.

2. The adamantane compound according to claim 1, wherein the adamantane compound is represented by the following general formula (1-A): 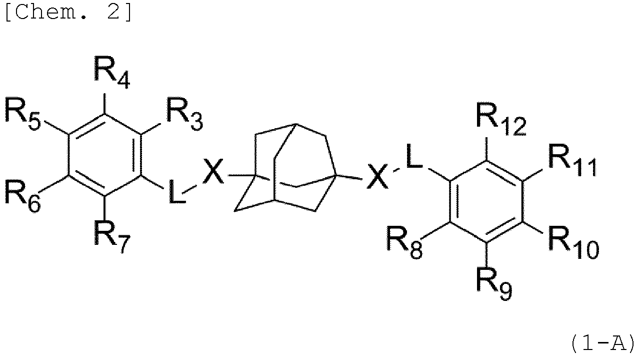 wherein X and L are as defined in the general formula (1), R₃ to R₁₂ may be the same or different, and represent a hydrogen atom, a halogen atom, a cyano group, a substituted or unsubstituted linear or branched alkyl group having 1 to 3 carbon atoms, a substituted or unsubstituted linear or branched alkyloxy group having 1 to 3 carbon atoms, or a substituted or unsubstituted aromatic hydrocarbon group.

3. The adamantane compound according to claim 1 or 2, wherein the adamantane compound is represented by the following general formula (1-B): 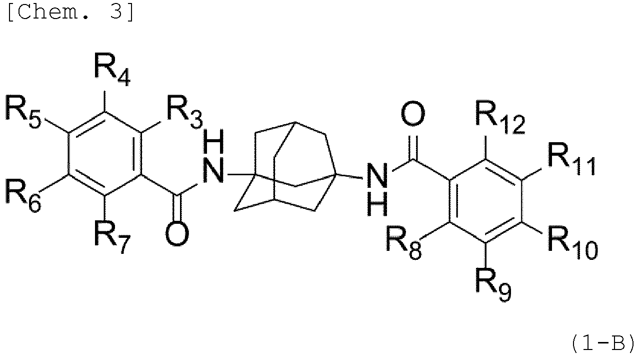 wherein R₃ to R₁₂ are as defined in the general formula (1-A).

4. The adamantane compound according to claim 1 or 2, wherein the adamantane compound is represented by the following general formula (1-C): 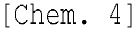 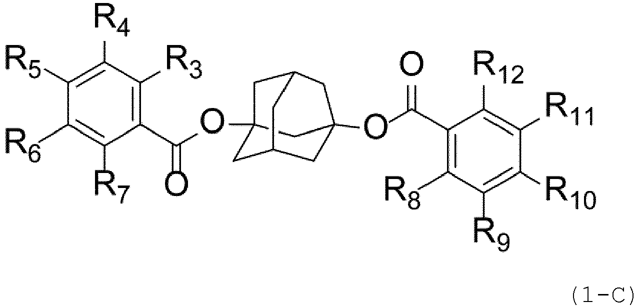 wherein R₃ to R₁₂ are as defined in the general formula (1-A).

5. The adamantane compound according to claim 1 or 2, wherein the adamantane compound is represented by the following general formula (1-D): 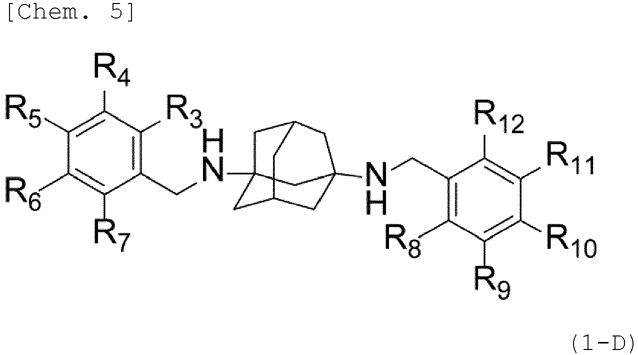 wherein R₃ to R₁₂ are as defined in the general formula (1-A).

6. The adamantane compound according to claim 1 or 2, wherein the adamantane compound is represented by the following general formula (1-E): 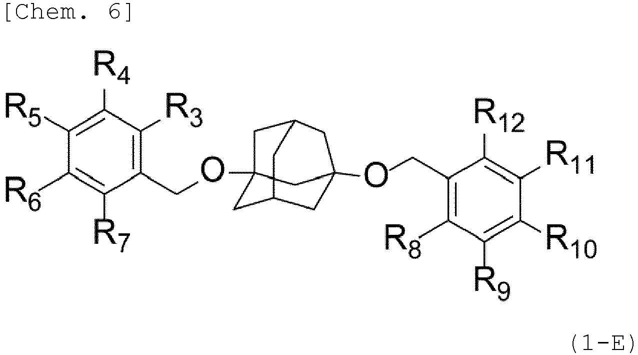 wherein R₃ to R₁₂ are as defined in the general formula (1-A).

7. An organic thin film comprising the adamantane compound according to any one of claims 1 to 6, **characterized by** having a refractive index in a wavelength range of 400 nm to 700 nm of 1.60 or less.

8. An organic electroluminescent device comprising at least an anode electrode, a hole transport layer, a light emitting layer, an electron transport layer, a cathode electrode, and a capping layer in this order, **characterized in that** the capping layer is the organic thin film according to claim 7.

9. An organic electroluminescent device comprising at least an anode electrode, a hole transport layer, a light emitting layer, an electron transport layer, a cathode electrode, and a capping layer in this order, **characterized in that** the capping layer has a two-layer structure of a first capping layer and a second capping layer, and the first capping layer is the organic thin film according to claim 7.

10. The organic electroluminescent device according to claim 9, **characterized in that** a difference between the refractive index of the first capping layer and the refractive index of the second capping layer ([the refractive index of the second capping layer] - [the refractive index of the first capping layer]) is 0.2 or more.

11. An electronic apparatus or an electronic device comprising a pair of electrodes and at least one organic layer sandwiched therebetween, **characterized in that** the adamantane compound according to any one of claims 1 to 6 is used for the organic layer as a constituent material thereof.
